# EUROPEAN PATENT APPLICATION

(11) **EP 2 929 885 A1**
(43) Date of publication of application: **14.10.2015**
(21) Application number: 15163028.2
(22) Date of filing: 09.04.2015
(51) Int. Cl.: A61K 31/4439, A61K 31/5377, A61K 9/20, A61K 9/24, A61K 9/00, A61P 7/02

(54) **PHARMACEUTICAL COMBINATIONS OF RIVAROXABAN AND PROTON PUMP INHIBITORS**

(30) Priority: 11.04.2014 TR 201404232; 10.09.2014 TR 201410626
(71) Applicant: Sanovel Ilac Sanayi ve Ticaret A.S., 34460 Istanbul (TR)
(72) Inventor: Türkyilmaz, Ali, 34460 Istanbul (TR); Yelken, Gülay, 34460 Istanbul (TR)
(74) Representative: Sevinç, Erkan

(57) **Abstract**

This invention is a novel pharmaceutical composition comprising rivaroxaban or a pharmaceutically acceptable salt thereof in combination with a proton pump inhibitor for use in the anticoagulant treatment with preventing or reducing the risk of a gastrointestinal disorder.

## Description

### Field of Invention

This invention is a novel pharmaceutical composition comprising rivaroxaban or a pharmaceutically acceptable salt in a combination with a proton pump inhibitor and at least one pharmaceutically acceptable excipient.

More specifically, this invention relates to pharmaceutical composition comprising rivaroxaban or a pharmaceutically acceptable salt thereof in combination with a proton pump inhibitor for use in the anticoagulant treatment with preventing or reducing the risk of a gastrointestinal disorder administrated by oral, parenteral, intramuscular or topical route.

### Background of Invention

Anticoagulant drugs target various procoagulant factors in the coagulation pathway. Presently there are numerous anticoagulant drugs which are widely available. One of them is direct factor Xa inhibitors which plays a central role in the cascade of blood coagulation by inhibiting factor Xa directly. In an exemplary embodiment, the Direct factor Xa inhibitors is a member selected from rivaroxaban, apixaban, edoxaban, betrixaban, TAK-442, darexaban, and pro-drug s thereof.

Rivaroxaban (Formula I), which is already known from WO0147919, is an anticoagulant and the first orally active direct factor Xa inhibitor. Rivaroxaban is used to treat deep vein thrombosis (DVT) and pulmonary embolism (PE). It is also used to help prevent strokes or serious blood clots in people who have atrial fibrillation.

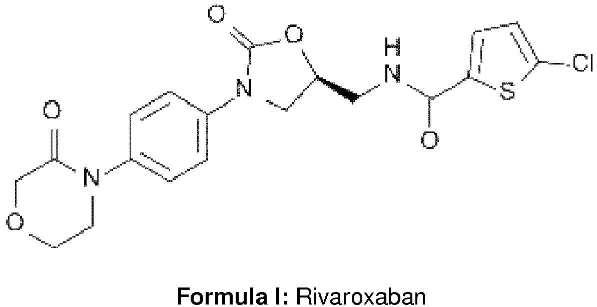

Rivaroxaban , which is commercially available under the trade name Xarelto®, is disclosed in US7157456 (B2), US7585860 (B2), US7592339 (B2).

However, rivaroxaban is associated with side effects. The administration of antitcoagulant agents, such as rivaroxaban, have been associated with gastrointestinal disorders such as ulcers and gastrointestinal bleeding. In addition, the administration of rivaroxaban may make some patients more susceptible to the ulcerogenic effects of ulcerogenic stimuli. It appears that a major factor contributing to the development of these gastrointestinal disorders is the presence of acid in the stomach and upper small intestine. While the mechanisms associated with ulcers and gastrointestinal bleeding are not entirely known, there are many causes of ulcers, including stress, alcohol irritation, Helicobacter pylori infection, and the side effects of non-steroid anti-inflammatory drugs. Patients in need of long term anticoagulant drug therapy often may interrupt or not receive such therapy due to gastrointestinal disorders, and as a result, patients are deprived of beneficial anticoagulant drug therapy. There is a need for oral pharmaceutical combination formulations to reduce or eliminate the gastrointestinal disorders associated with use of anticoagulant drugs.

Proton pump inhibitors (PPI) are acid labile compounds useful for inhibiting gastric acid secretion. The problems arisen from this acid labile characteristic of these compounds is overcome by providing a formulation that dissolves in the intestines rather than the acidic enviroment of the stomach.

Using an antacid or an alkaline excipient with a PPI in the formulation is one method used in the art to reduce the acidity of the gastrointestinal environment. Another one is to provide an enteric coating for the composition comprising the acid labile PPI.

It is known in the state of art that the enteric polymers with acidic nature are not compatible with the proton pump inhibitors and the alkaline excipients used in the formulation. To overcome this problem an inert intermediate layer may be introduced, such as in the prior art. In US4,853,230 and WO96/24338 enteric coated formulations with an alkaline active ingredient containing core and a separating layer are described.

Another role of the enteric coating is to provide a modified release. The delivery of the active ingredient to specific sites in the gastrointestinal tract is achieved by enteric coatings. These polymers can also prolong or extend the amount of active ingredient released from the dosage form.

Lansoprazole is a proton pump inhibitor (PPI) which inhibits the stomach's production of gastric acids. Lansoprazole is a racemate 1:1-mixture of the enantiomers dexlansoprazole and levolansoprazole. Dexlansoprazole is an enantiomerically pure active ingredient of a commercial drug as a result of the enantiomeric shift.

In prior art, there are many patents including benzimidazoles such as lansoprazole and its R-enantiomer, dexlansoprazole in several different pharmaceutical compositions. Crystal form of R-Iansoprazole is described in EP1129088.

One of the the active ingredient from the group of PPI, dexlansoprazole is the R-enantiomer of lansoprazole which inhibits gastric acid secretion (a proton pump inhibitor). Its chemical name is (+)-2-[(R)-{[3-methyl-4-(2,2,2-trifluoroethoxy)pyridin-2-yl] methyl} sulfinyl]-1 H-benzimidazole and its chemical structure is shown in the Formula II.

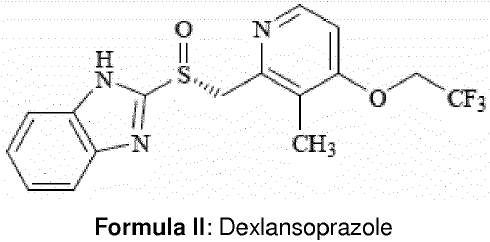

Delayed release capsule form of dexlansoprazole is in the market and it is administered orally in a therapeutic dose of 30 mg and 60 mg.

As other benzimidazole compounds, dexlansoprazole has also poor stability and is unstable to acidic medium, humidty, light and sensitive to heating. When orally administrated, it may not be able to sufficient activity since it is decomposed by gastric acid and the like. Thus, several problems occur in formulating such compound into oral pharmaceutical dosage forms because of the acidic environmental of the stomach. In particular, it will be rapidly decomposed and change colour under moist conditions or in acidic to neutral aqueous solution.

Another one from the group of PPI is the compound 5-methoxy-2-[[(4-methoxy-3,5-dimethyl-2-pyridinyl)methyl]sulfinyl]-1H-benz imidazole, having the generic name omeprazole, and therapeutically acceptable alkaline salts thereof are described in U.S. Pat. No. 4,255,431 to Junggren et al., EP 5129 and EP 124 495, respectively. Omeprazole and structurally similar sulfoxide compounds are known inhibitors of gastric acid secretion and are used as anti-ulcer agents. The sulfur atom of the sulfoxide group in asymmetrically substituted sulfoxides is chiral. Therefore, omeprazole and related sulfoxides exhibit optical isomerism at the sulfur atom of the sulfoxide. In fact, omeprazole exists as a pair of enantiomers; the S (-) enantiomer is referred to as esomeprazole.

Esomeprazole is a proton pump inhibitor that suppresses gastric acid secretion by specific inhibition of the H+/K+-ATPase in the gastric parietal cell. The S- and R-isomers of omeprazole are protonated and converted in the acidic compartment of the parietal cell forming the active inhibitor, the achiral sulphenamide. By acting specifically on the proton pump, esomeprazole blocks the final step in acid production, thus reducing gastric acidity. This effect is dose-related up to a daily dose of 20 to 40 mg and leads to inhibition of gastric acid secretion. Its chemical name is (S)-5-methoxy-2-[(4-methoxy-3,5-dimethylpyridin-2-yl) methylsulfinyl]-3H-benzoimidazole and its chemical structure is shown in the Formula III. Certain salts of single enantiomers of omeprazole and their preparation are disclosed in WO 94/27988. These compounds have improved pharmacokinetic and metabolic properties which will give an improved therapeutic profile such as a lower degree of interindividual variation. WO 96/02535 discloses a process for the preparation of the single enantiomers of omeprazole and salts thereof.

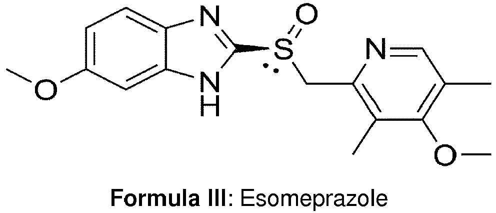

Pharmaceutical compositions that include one or more compounds obtained by the process aspect of the invention are also provided. In particular, in one variant, a pharmaceutical composition that includes the amorphous esomeprazole produced as described in U.S. Pat. No. 2004077869. The more preferred oral solid preparation is a tablet. A tablet may be prepared by direct compression, wet granulation, or molding, of the amorphous form of esomeprazole with a carrier and other excipients in a manner known to those skilled in the art. The amorphous form of esomeprazole described U.S. Pat. No. 2004077869 may be formulated into typical disintegrating tablet, or into a controlled or extended release dosage forms. Examples of suitable modified release formulation vehicles are disclosed in U.S. Pat. Nos. 3,845,770; 3,916,899; 3,536,809; 3,598,123; and 4,008,719. WO 96/01623 discloses suitable tableted dosage forms of for instance magnesium salts R- and S-omeprazole.

As described above, the major factor contributing to the development of gastrointestinal disorders is the presence of acid in the stomach and upper small intestine. Therefore, the main object of the invention is administering to a patient an oral dosage form including a combination of an anticoagulant agent and a proton pump inhibitor which can reduce a major factor contributing to the development of gastrointestinal disorder in patients. According to the main object, the pharmaceutical compositions of this invention is for the prevention gastrointestinal tract from side effects associated with anticoagulant therapy and provide greater anticoagulant effect with the results in a lower incidence of side effects. According to prior inventions rivaroxaban in combination with proton pump inhibitors in particular dexlansoprazole or esomeprazole have an additive preventive effect in relief of gastrointestinal disorder and provide greater anticoagulant effect with the results in a lower incidence of side effects.

### Detailed description of the invention

The present invention relates to a pharmaceutical composition comprising rivaroxaban in a combination with a proton pump inhibitor or a pharmaceutically acceptable salt, solvate, hydrate, enantiomer, pro-drug or polymorph thereof and at least one pharmaceutically acceptable excipient

The main object of the present invention is to provide new pharmaceutical compositions comprising a specific direct factor Xa inhibitor such as rivaroxaban in combination with proton pump inhibitors selected from the group comprising cinprazole, dexlansoprazole, disuprazole, esaprazole, esomeprazole, fuprazole, ilaprazole, lansoprazole, leminoprazole, levolansoprazole, nepaprazole, nilprazole, omeprazole, pantoprazole, picoprazole, pumaprazole, rabeprazole, saviprazole, tenatoprazole, timoprazole, ufiprazole or other benzimidazole derivatives or a pharmaceutically acceptable salt, solvate, hydrate, enantiomer, pro-drug or polymorph thereof as effective components which overcomes the above described problems in prior art and have additive advantages over them.

According to the formulation of the present invention it is desired to provide a dosage form comprising in combination a therapeutically effective amount of an anticoagulant agent, specifically rivaroxaban and a proton pump inhibitor specifically dexlansoprazole or esomeprazole which overcomes above described problems. The main challenges when combining those molecules in the same pharmaceutical form are:
(a) to guarantee the psycho-chemical compatibility between those different active ingredients and/or between the active ingredients and the excipients used; and
(b) to insure the therapeutical compatibility between those active ingredients regarding their stability characteristics.

When rivaroxaban is combined with PPI specifically dexlansoprazole or esomeprazole in order to minimize or prevent the side effects of the rivaroxaban , these two drug substances must be released, dissolved and absorbed harmoniously. For the efficiency of the formulation, an easy dissolution of both drug substances at a desired time is an important factor. In order to minimize or prevent the side effects of rivaroxaban ; it is very important for the molecule, in which the molecule is used in combination with the PPIs to release both drug substances at the desired time.

The product in this invention is used for protecting the gastrointestinal tract from side effects associated with anticoagulant therapy using the oral dosage forms described herein. The invention also relates to protect the gastrointestinal tract from side effects associated with anticoagulant therapy by combination an anticoagulant agent and a proton pump inhibitor. These and other features of the present invention are set forth herein more detailed.

In one embodiment, the proton pump inhibitor is preferably lansoprazole or omeprazole or an acceptable salt, solvate, hydrate, enantiomer, pro-drug or polymorph thereof.

In one embodiment, the pharmaceutical composition comprises rivaroxaban and lansoprazole or a pharmaceutically acceptable salt, solvate, hydrate, enantiomer, pro-drug or polymorph thereof. In a preferred embodiment, lansoprazole is present in an amount of between 15 mg and 60 mg. In a more preferred embodiment, lansoprazole is present in an amount of between 15 mg and 30 mg.

In one embodiment, the pharmaceutical composition comprises rivaroxaban in a combination with an enantiomer of lansoprazole which is dexlansoprazole. In a preferred embodiment, dexlansoprazole is present in an amount of between 10 mg and 75 mg. In a more preferred embodiment, dexlansoprazole is present in an amount of 20 mg.

In one embodiment, the pharmaceutical composition of the present invention comprises rivaroxaban and omeprazole or a pharmaceutically acceptable salt, solvate, hydrate, enantiomer, pro-drug or polymorph thereof. According to this embodiment, omeprazole is present in an amount of between 10 mg and 75 mg. In a more preferred embodiment, omeprazole is present in an amount of 20 mg.

In one embodiment, the pharmaceutical composition comprises rivaroxaban in a combination with an enantiomer of omeprazole which is esomeprazole. In a preferred embodiment, esomeprazole is present in an amount of between 10 mg and 75 mg. In a more preferred embodiment, esomeprazole is present in an amount of 20 mg.

In a preferred embodiment, rivaroxaban is present in an amount of between 1 and 250 mg. In a more preferred embodiment, rivaroxaban is preferably in an amount of between 5 and 100 mg.

In a preferred embodiment rivaroxaban present in an amount of between 5 and 100 mg and the dexlansoprazole or a pharmaceutically acceptable salt, solvate, hydrate, enantiomer, pro-drug or polymorph thereof is present in an amount of between 10 and 75 mg.

In another preferred embodiment rivaroxaban present in an amount of between 5 and 100 mg and the esomeprazole or a pharmaceutically acceptable salt, solvate, hydrate, enantiomer, pro-drug or polymorph thereof is present in an amount of between 10 and 75 mg.

According to main object of the invention, pharmaceutical formulations of rivaroxaban in a combination with a proton pump inhibitor have a good stability and have improved manufacturing process that is achieved in a technologically simple way according to prior art which overcomes the above described problems.

Yet another object of the present invention is to provide an improved process which is simple, cost-effective and time saving for preparing the pharmaceutical composition of rivaroxaban and one or more PPIs specially esomeprazole and dexlansoprazole.

In another embodiment, the oral formulations described herein can be used to treat, prevent or reduce the risk of almost any physiological disorder for which anticoagulant agents are indicated. The methods and formulations provided herein can be administered to any subject in need of therapy including, without limitation, humans, including patients, companion animals, including, but not limited to dogs, cats, ferrets, and birds, food-source animals, including, but not limited to cows, pigs, and sheep, and zoo animals, such as monkeys and other primates, and other similar animal species.

Another embodiment of this invention, the pharmaceutical composition is in the form of solid, liquid or semisolid dosage form.

In one embodiment, these pharmaceutical combinations are administrated oral, parenteral, intranasal, sublingual, transdermal, transmucosal, ophthalmic, intravenous, pulmonary, intramuscular or rectal administration, and preferably for oral administration. According to this embodiment of the invention, said pharmaceutical composition may be formulated with suitable pharmaceutical diluents, excipients or carriers, suitably selected with respect to a dosage form for oral administration. Examples of dosage forms include compressed tablets, coated tablets, uncoated tablets, multilayer tablets, buccal tablets, sublingual tablets, tablet in tablets, in-lay tablets, effervescent compositions, effervescent tablets, immediate release tablets, modified release tablets, film-coated tablets, orally disintegrating tablets, gastric disintegrating tablets, pills, capsules, hard or soft gelatin capsules, powders, mini tablets, pellets, coated bead systems, granules, microspheres, ion exchange resin systems, sterile solutions or suspensions, sterile ocular solutions, aerosols, sprays, drops, ampoules, suppositories, ocular systems, parenteral systems, creams, gels, ointments, dragees, sachets, films, orally administrable films, solutions, solids; elixirs, tinctures, suspensions, syrups, colloidal dispersions, dispersions, emulsions and thereof.

Another object of the invention is to provide a pharmaceutical composition for use in anticoagulant treatment and treating deep vein thrombosis (DVT) and pulmonary embolism (PE) and protecting the gastrointestinal tract from side effects associated with anticoagulant therapy.

In a preferred embodiment said pharmaceutical composition is formulated preferably in the form of tablet or capsule or multilayer tablet comprising rivaroxaban pellets and proton pump inhibitor pellets

According to the pharmaceutical incompatibility problem, the present invention provides a pharmaceutical composition comprising a barrier layer which is present in the middle in such a way that it separates rivaroxaban and one of the proton pump inhibitors layers and the rivaroxaban and PPI layers are present at the outer or inner part of the barrier layer. By the virtue of the barrier layer, interaction between the rivaroxaban and PPI molecules is prevented. Moreover, another important reason is that the formulations of the active substances with different release properties can be provided in the same form with barrier layer. In order to minimize gastric damage, enteric coated formulations such that controlled release thereof will be performed with the combination with a barrier layer.

In one embodiment of the invention, the bilayer or multilayer tablet may comprise optionally a barrier layer between the two layers wherein the barrier layer is selected from the group comprising corn starch, lactose, sugar alcohol (D-mannitol, erythritol, etc.), lowsubstituted hydroxypropyl cellulose, hydroxypropyl cellulose, hydroxypropyl methylcellulose, polyvinylpyrrolidone, polyvinyl alcohol, methylcellulose, hydroxyethyl methylcellulose or mixtures thereof. In a preferred embodiment of the present invention said barrier layer comprising preferably cellulosic polymers. Thus, the multilayer tablet according to the present invention has two or more layers comprising the rivaroxaban and PPI molecules seperately and it is characterized in releasing these molecules together and rapidly. The multilayer tablet according to the present invention is formulated in such a way that it increases the pH of the stomach by the dissolution of PPI after the dissolution of rivaroxaban that provides less gastrointestinal damage.

According to the challenges mentioned above the selection of the excpients thus very important. According to this embodiment, one or more pharmaceutically acceptable excipient is selected from the group comprising buffering agents, stabilizers, binders, diluents, dispersing agents, lubricants, glidants, disintegrants, plasticizers, preservatives, sweeteners, flavoring agents, coloring agents, wetting agents, emulsifiers or mixtures thereof.

Suitable buffering agents may comprise but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate or mixtures thereof.

Suitable stabilizers may comprise but not limited to citric acid, fumaric acid, tartaric acid, sodium citrate, sodium benzoate, sodium dihydrogen phosphate, calcium carbonate, magnesium carbonate, arginine, lysine, meglamine, tocopherol, butylhydroxyanisole (BHA), butylhydroxytoluene (BHT), ascorbic acid, gallic acid esters or the mixtures thereof, and preferably, citric acid, fumaric acid, arginine or mixtures thereof.

Suitable binders may include but not limited to polyvinylpyrrolidone, polyethylene glycol, polyvinyl alcohol, starch, pregelatinized starch, glucose, glucose syrup, natural gums, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, proteins like gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable diluents may comprise but not limited to microcrystalline cellulose, mannitol, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

Suitable dispersing agents may comprise but not limited to calcium silicate, magnesium aluminum silicate or mixtures thereof.

Suitable lubricants may comprise but not limited to magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable glidants may comprise but not limited to talc, aluminium silicate, colloidal silica, starch or mixtures thereof.

Suitable disintegrants may comprise but not limited to cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof.

Suitable plasticizers may comprise but not limited to polyethylene glycols of different molecular weights, propylene glycol or mixtures thereof.

Suitable preservatives may comprise but not limited to methyl paraben, propyl paraben and their salts (such as sodium, potassium), sodium benzoate, citric acid, benzoic acid, butylated hydroxytoluene or butylated hydroxyanisole or mixtures thereof.

Suitable sweeteners may comprise but not limited to aspartame, potassium acesulfame, sodium saccharinate, neohesperidine dihydrochalcone, sucralose, saccharin, sugars such as sucrose, glucose, lactose, fructose or sugar alcohols such as mannitol, sorbitol, xylitol, erythritol or mixtures thereof.

Suitable flavorings may comprise but not limited to menthol, peppermint, cinnamon, chocolate, vanillin or fruit essences such as cherry, orange, strawberry, grape, black currant, raspberry, banana, red fruits, wild berries or mixtures thereof.

Suitable coloring agents may comprise but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

In a preferred embodiment of the present invention, said pharmaceutical composition is in the form of tablet or capsule or multilayer tablet comprising of rivaroxaban pellets and proton pump inhibitor pellets which are separate pellets or bilayer or multilayer pellets.

In a preferred embodiment of the present invention said rivaroxaban pellets comprising preferably methacrylic acid-ethyl acrylate copolymer (Eudragit EPO) or polyvinyl alcohol (PVA) or lactose monohydrate or sugar pellet or polyvinylpyrrolidone (PVP) or dibasic calcium phosphate (DCP) or mixtures thereof as pharmaceutically acceptable excipients

In a preferred embodiment of the present invention said proton pump inhibitor pellets comprising preferably sugar pellet or mannitol or sodium starch glycolate or magnesium carbonate or povidone (K30) or sucrose or poloxamer (407) or polyvinyl alcohol (PVA) or methacrylic acid- ethyl acrylate copolymer or talc or triethyl citrate or simethicone emulsion or dibasic calcium phosphate (DCP) or polyvinylpyrrolidone (PVP) or eudragit (L100-55) or kollicoat (MAE 30DP) or polyvinyl acetate (kollidon SR) or mixtures thereof as pharmaceutically acceptable excipients

When these compounds are formulated into pharmaceutical preparations for oral administration, they require special techniques to avoid contact of PPIs with gastric acid of the stomach. One technique most commonly used is to coat these compounds, or its granules or pellets, with an enteric coating. However, the material used in enteric coatings itself is acidic, which can cause the decomposition of the compound. Such decomposition occurs even during the enteric coating process, which results in the coloration of the surface of the drug-containing core.

Enteric films do not show high flexibility so that compression stress can yield rupturing of the film. It is therefore necessary to use a tableting technique that endorses the compression strain and maintains the acid resistance of the formulation after compression of the granules. Therefore caution is needed to be taken while compressing the tablets especially to form bilayer dosage form.

In other embodiments, the enteric coating surrounds the anticoagulant agent in the oral dosage form. In still another embodiment, the enteric coating surrounds both the proton pump inhibitor and the anticoagulant agent in the oral dosage form. In some embodiments, the enteric coating surrounds essentially all of the proton pump inhibitor. In other embodiments, the enteric coating surrounds essentially all of the anticoagulant agent. In still other embodiments, the enteric coating surrounds essentially all of both the proton pump inhibitor and the anticoagulant agent in the oral dosage form. In other embodiments, the oral dosage form does not include an enteric coating. In other embodiments, the oral dosage form is substantially free of an enteric coating.

In a preferred embodiment of the present invention said proton pump inhibitor pellets comprises enteric coating in an amount of 1% to 20% (w/w) of the total weight of the pellets preferably in an amount of 2% to 10% (w/w) wherein the enteric coating dissolves at pH 5.5 or higher.

According to this embodiment, the enteric polymers of the enteric coating layer composition can be selected from the group comprising of cellulose acetate phthalate, cellulose acetate succinate, hydroxpropyl cellulose, hydroxpropyl cellulose phthalate, hydroxpropyl ethylcellulose, hydroxpropyl ethylcellulose phthalate, hydroxyl propyl methyl cellulose, hydroxyl propyl methyl cellulose phthalate, hydroxyl propyl methyl cellulose acetate succinate, hydroxyethyl cellulose phthalate, methylcellulose phthalate, polyvinyl acetate, polyvinyl acetate phthalate, polyvinylacetate hydrogen phthalate, cellulose ester phthalates, cellulose ether phthalates, sodium cellulose acetate phthalate, starch, starch acid phthalate, cellulose acetate butyrate, cellulose acetate maleate, cellulose acetate trimellitate, cellulose acetate propionate, cellulose acetate phthalate, styrene maleic acid dibutyl phthalate copolymer, styrene maleic acid polyvinyl acetate phthalate copolymer, ammonium methacrylate copolymers, hydrogels, carboxyvinyl polymer, sodium alginate, sodium carmellose, calcium carmellose, polyvinyl alcohol, polyvinyl alcohol-polyethylene glycol mixtures, gelatine, polyvinylpyrrolidone, microcrystalline cellulose, collagen, ethyl cellulose, carboxymethyl cellulose, carnauba wax, pectin, carbomer, poloxamer, polyachrylamide, aluminium hydroxide, bentonit, laponit, setostearyl alcohol, polyoxyethylen-alkyl ethers, hyaluronic acid, guar gum, cocoa oil, paraffin, hydrogenated vegetable oil, cetyl alcohol, stearyl alcohol, stearic acid, xanthane gum, shellac or mixtures thereof.

According to the pharmaceutical compositions of the invention may be prepared by conventional technology well known to those skilled in the art such as direct compression, dry granulation, wet granulation and the like. During direct compression, active agent and excipients are mixed, sieved and compressed into dosage forms. During wet granulation, the ingredients are mixed and granulated with a granulation liquid. The granulation process provides agglomerates with a desired homogeneity. The mixture is dried and sieved and optionally mixed with additional excipients. Finally, it is compressed into dosage forms. In addition, this novel pharmaceutical formulation is produced by various technologies such as fluidized bed granulation technique or extrusion/spheronization or spray drying and lyofilization.

### Examples:

### Example-1: Rivaroxaban + PPI tablet or capsule

### Rivaroxaban pellets:

%5.0 - 95 Rivaroxaban
% 0.05 - 20 Polyvinyl alcohol (PVA) or isopropyl alcohol (IPA)
% 0.05 - 20 Sodium alginate
% 0.5 - 40 Methacrylic acid-Ethyl acrylate copolymer (Eudragit EPO) or PVA

### PPI pellets:

%2.0- 30 Esomeprazole or Dexlansoprazole
%5.0- 90 Sugar Pellet
%5.0- 90 Mannitol
%0.05- 10 Sodium starch glycolate
%0.5- 60 Magnesium carbonate
%0.1- 30 Povidone K30
%0.05- 30 Sucrose
%0.05- 30 Poloxamer 407
%0.05- 50 Polyvinyl alcohol (PVA)
%0.5- 40 Methacrylic acid- Ethyl acrylate copolymer (1:1) dispersion 30%
%0.5- 30 Talc
%0.02- 20 Triethyl citrate
%0.001- 5 Simethicone emulsion

### Other Excipients:

%0.1 - 1.0 Silicon dioxide
%0.1 - 3.0 Magnesium stearate

**Production process of Rivaroxaban pellets:** Rivaroxaban is dissolved in alcoholic Methacrylic acid-Ethyl acrylate copolymer (Eudragit EPO) or Polyvinyl alcohol (PVA) solution. PVA or IPA (isopropyl alcohol) solution is sprayed onto this mixture and pellets produced by fluidized bed pelletizing technique. The pellets are coated with sodium alginate in fluidized bed so rivaroxaban pellets are manufactured.

**Production process of PPI pellets:** Esomeprazole/Dexlansoprazole, mannitol, sodium starch glycolate, magnesium carbonate, povidone K30 and sucrose are mixed together. Poloxamer 407 and alcoholic PVA solution are added to this mixture. This solution/dispersion is sprayed onto the sugar pellets. Methacrylic acid:Ethyl acrylate copolymer (1:1) dispersion (30%) is prepared with talc, triethyl sitrate, simethicone emulsion. This pellets are coated by spraying this mixture.

The rivaroxaban and PPI pellets first mixed with silicon dioxide and then with magnesium stearate. Finally, the pellets are pressed as tablets or filled into the capsules.

### Example-2: Rivaroxaban + PPI tablet or capsule

### Rivaroxaban pellets:

%5.0 - 95 Rivaroxaban
%0.5 - 40 Methacrylic acid-Ethyl acrylate copolymer (Eudragit EPO) or Polyvinyl alcohol (PVA)
%5.0 - 90 Lactose monohydrate

### PPI pellets:

%2.0 - 30 Esomeprazole orDexlansoprazole
%5.0 - 90 Dibasic calcium phosphate (DCP)
%0.1 - 30 Polyvinylpyrrolidone (PVP)
%0.5 - 40 Polyvinyl alcohol (PVA)

### Other Excipients:

%0.1 - 1.0 Silicon dioxide
%0.1 - 3.0 Magnesium stearate

**Production process of Rivaroxaban pellets:** Rivaroxaban and lactose monohydrate are mixed together. Alcoholic Methacrylic acid-Ethyl acrylate copolymer (Eudragit EPO) or Polyvinyl alcohol (PVA) solution is sprayed onto this mixture and pellets produced by fluidized bed pelletizing technique.

**Production process of PPI pellets:** Esomeprazole/Dexlansoprazole and DCP and PVP are blended, then by spraying PVA alcoholic/hydroalcoholic solution a wet mass is formed, Pellets produced from this wet mass by extrusion/spheronization pelletizing technique. The pellets are coated by PVA solution in fluidized bed.

The rivaroxaban and PPI pellets first mixed with silicon dioxide and then with magnesium stearate. Finally, the pellets are pressed as tablets or filled into the capsules.

### Example-3: Coated Core Pelletization (Rivaroxaban pellets+ PPI pellets)

### Rivaroxaban pellets

%5.0 - 95 Rivaroxaban
%5 - 90 Sugar pellet
%0.1 - 30 Polyvinylpyrrolidone (PVP)
%0.5 - 40 Methacrylic acid-Ethyl acrylate copolymer (Eudragit EPO or Polyvinyl alcohol (PVA)

### PPI pellets

%2.0 - 30 Esomeprazole or Dexlansoprazole
%5.0 - 90 Sugar pellet
%0.1 - 30 Polyvinylpyrrolidone (PVP)
%0.5 - 40 Methacrylic acid-Ethyl acrylate copolymer (Eudragit L100-55 or Kollicoat MAE 30DP)
%0.5 - 40 Polyvinyl acetate (Kollidon SR)

### Other Excipients

%0.1 - 1.0 Silicon dioxide
%0.1 - 3.0 Magnesium stearate

**Production process of Rivaroxaban pellets:** Alcoholic solution/dispersion is prepared by adding rivaroxaban and PVP. Sugar pellets are coated with this solution/dispersion. The sugar pellets which were coated with the active ingredient are coated with Methacrylic acid-Ethyl acrylate copolymer (Eudragit EPO or Polyvinyl alcohol (PVA) and then with PVA solution so rivaroxaban pellets are manufactured.

**Production process of PPI pellets:** Alcoholic solution/dispersion is prepared by adding Esomeprazole/Dexlansoprazole and PVP. Sugar pellets are coated with this solution/dispersion. The sugar pellets which were coated with the active ingredient are coated with Methacrylic acid-Ethyl acrylate copolymer (Eudragit L100-55 or Kollicoat MAE 30DP) and Polyvinyl acetate (Kollidon SR) and then with PVA solution so PPI pellets are manufactured.

Coated PPI pellets and rivaroxaban pellets are mixed. The pellets are first mixed with silicon dioxide and then with magnesium stearate. Finally, the pellets are pressed as tablets or filled into the capsules

### Example-4: Multilayer combination pellet (multilayer pellet production)

%2.0 - 30 Esomeprazole or Dexlansoprazole
%5.0 - 90 Sugar pellet
%0.1 - 30 Polyvinylpyrrolidone (PVP)
%0.5 - 40 Methacrylic acid-Ethyl acrylate copolymer (Eudragit L100-55 or Kollicoat MAE 30DP)
%0.5 - 40 Polyvinyl acetate (Kollidon SR)
%5.0 - 95 Rivaroxaban
%0.5 - 40 Methacrylic acid-Ethyl acrylate copolymer (Eudragit EPO) or Polyvinyl alcohol (PVA)
%5.0 - 90 Lactose monohydrate
% 0.05 - 20 Polyvinyl alcohol (PVA)
%0.1 - 1.0 Silicon dioxide
%0.1 - 3.0 Magnesium stearate

**Production process multilayer pellets:** Alcoholic solution/dispersion is prepared by adding Esomeprazole/Dexlansoprazole and PVP. Sugar pellets are coated with this solution/dispersion. The sugar pellets which were coated with the active ingredient are coated with Methacrylic acid-Ethyl acrylate copolymer (Eudragit L100-55 or Kollicoat MAE 30DP) and polyvinyl acetate (Kollidon SR) and then with PVA solution so PPI pellets are manufactured. Rivaroxaban and lactose monohydrate are mixed with alcoholic Methacrylic acid-Ethyl acrylate copolymer (Eudragit EPO) or Polyvinyl alcohol (PVA) solution. This mixture is sprayed onto PPI pellets so multilayer pellets are manufactured.

Finally the multilayer pellets are coated by PVA. The multilayer pellets first mixed with silicon dioxide and then with magnesium stearate. Finally, the pellets are pressed as tablets or filled into the capsules.

### Example-5: Multilayer combination pellet (multilayer tablet press)

### Rivaroxaban pellets :

%5.0- 95 Rivaroxaban (RIVAROXABAN)
%0.5- 40 Methacrylic acid-Ethyl acrylate copolymer (Eudragit EPO) or Polyvinyl alcohol (PVA)
%5.0 - 90 Lactose monohydrate
% 0.05 - 20 Polyvinyl alcohol (PVA)

### PPI pellets:

%2.0 - 30 Esomeprazole/Dexlansoprazole
%5.0 - 90 Sugar pellet
%0.1 - 30 Polyvinylpyrrolidone (PVP)
%0.5 - 40 Methacrylic acid-Ethyl acrylate copolymer (Eudragit L100-55 or Kollicoat MAE 30DP)
%0.5 - 40 Polyvinyl acetate (Kollidon SR)
%0.1 - 1.0 Silicon dioxide
%0.1 - 3.0 Magnesium stearate

**Production process of Rivaroxaban pellets:** Rivaroxaban and lactose monohydrate are mixed together. Alcoholic Methacrylic acid-Ethyl acrylate copolymer (Eudragit EPO) or Polyvinyl alcohol (PVA) solution is sprayed onto this mixture and pellets produced by fluidized bed pelletizing technique.

**Production process of PPI pellets:** Alcoholic solution/dispersion is prepared by adding Esomeprazole/Dexlansoprazole and PVP. Sugar pellets are coated with this solution/dispersion. The sugar pellets which were coated with the active ingredient are coated with Methacrylic acid-Ethyl acrylate copolymer (Eudragit L100-55 or Kollicoat MAE 30DP) and Polyvinyl acetate (Kollidon SR) and then with PVA solution so PPI pellets are manufactured.

The rivaroxaban and PPI pellets first mixed with silicon dioxide and then with magnesium stearate. Finally, the pellets are pressed by multilayer rotary tablet press machine. Or dry coating-intertwined tablets which have PPI pellets on the core are produced with special tablet press machine.

## Claims

1. A pharmaceutical composition comprising rivaroxaban in a combination with a proton pump inhibitor or a pharmaceutically acceptable salt, solvate, hydrate, enantiomer, pro-drug or polymorph thereof and at least one pharmaceutically acceptable excipient.

2. The pharmaceutical composition according to claim 1, wherein the proton pump inhibitor is selected from the group comprising cinprazole, dexlansoprazole, disuprazole, esaprazole, esomeprazole, fuprazole, ilaprazole, lansoprazole, leminoprazole, levolansoprazole, nepaprazole, nilprazole, omeprazole, pantoprazole, picoprazole, pumaprazole, rabeprazole, saviprazole, tenatoprazole, timoprazole, ufiprazole or a pharmaceutically acceptable salt, solvate, hydrate, enantiomer, pro-drug or polymorph thereof.

3. The pharmaceutical composition according to claim 2, wherein the proton pump inhibitor is preferably lansoprazole or omeprazole or an acceptable salt, solvate, hydrate, enantiomer, pro-drug or polymorph thereof.

4. The pharmaceutical composition according to claim 3, wherein said pharmaceutical composition comprising rivaroxaban and lansoprazole or a pharmaceutically acceptable salt, solvate, hydrate, enantiomer, pro-drug or polymorph thereof.

5. The pharmaceutical composition according to claim 4, wherein the enantiomer of lansoprazole is dexlansoprazole.

6. The pharmaceutical composition according to claim 3, wherein said pharmaceutical composition comprising rivaroxaban and omeprazole or a pharmaceutically acceptable salt, solvate, hydrate, enantiomer, pro-drug or polymorph thereof.

7. The pharmaceutical composition according to claim 6, wherein the enantiomer of omeprazole is esomeprazole.

8. The pharmaceutical composition according to claim 1, wherein rivaroxaban is present in an amount of between 1 and 250 mg.

9. The pharmaceutical composition according to claim 8, wherein rivaroxaban is preferably in an amount of between 5 and 100 mg.

10. The pharmaceutical composition according to claim 5, wherein rivaroxaban present in an amount of between 5 and 100 mg and the dexlansoprazole or a pharmaceutically acceptable salt, solvate, hydrate, enantiomer, pro-drug or polymorph thereof is present in an amount of between 10 and 75 mg.

11. The pharmaceutical composition according to claim 7, wherein rivaroxaban present in an amount of between 5 and 100 mg and the esomeprazole or a pharmaceutically acceptable salt, solvate, hydrate, enantiomer, pro-drug or polymorph thereof is present in an amount of between 10 and 75 mg.

12. The pharmaceutical composition according to claim 1, wherein said pharmaceutical composition is in the form of solid, liquid or semisolid dosage form.

13. The pharmaceutical composition of any preceding claims, wherein said pharmaceutical composition is formulated as compressed tablets, coated tablets, uncoated tablets, multilayer tablets, buccal tablets, sublingual tablets, tablet in tablets, in-lay tablets, effervescent compositions, effervescent tablets, immediate release tablets, modified release tablets, film-coated tablets, orally disintegrating tablets, gastric disintegrating tablets, pills, capsules, hard or soft gelatin capsules, powders, mini tablets, pellets, coated bead systems, granules, microspheres, ion exchange resin systems, sterile solutions or suspensions, sterile ocular solutions, aerosols, sprays, drops, ampoules, suppositories, ocular systems, parenteral systems, creams, gels, ointments, dragees, sachets, films, orally administrable films, solutions, solids; elixirs, tinctures, suspensions, syrups, colloidal dispersions, dispersions, emulsions and thereof.

14. The pharmaceutical composition according to claim 13, wherein said pharmaceutical composition is formulated preferably in the form of tablet or capsule or multilayer tablet comprising rivaroxaban pellets and proton pump inhibitor pellets.

15. The pharmaceutical composition of any preceding claims, wherein the one or more pharmaceutically acceptable excipients are selected from the group comprising buffering agents, stabilizers, binders, diluents, dispersing agents, lubricants, glidants, disintegrants, plasticizers, preservatives, sweeteners, flavoring agents, coloring agents, wetting agents, emulsifiers or mixtures thereof.

16. The pharmaceutical composition according to claim 15, wherein said pharmaceutical composition is in the form of tablet or capsule or multilayer tablet comprising of rivaroxaban pellets and proton pump inhibitor pellets which are separate pellets or bilayer or multilayer pellets.

17. The pharmaceutical composition according to claim 16, wherein said rivaroxaban pellets comprising preferably methacrylic acid-ethyl acrylate copolymer or polyvinyl alcohol (PVA) or lactose monohydrate or sugar pellet or polyvinylpyrrolidone (PVP) or dibasic calcium phosphate (DCP) or mixtures thereof.

18. The pharmaceutical composition according to claim 16, wherein said proton pump inhibitor pellets comprising preferably sugar pellet or mannitol or sodium starch glycolate or magnesium carbonate or povidone or sucrose or poloxamer or polyvinyl alcohol (PVA) or methacrylic acid- ethyl acrylate copolymer or talc or triethyl citrate or simethicone emulsion or dibasic calcium phosphate (DCP) or polyvinylpyrrolidone (PVP) or or polyvinyl acetate or mixtures thereof.

19. The pharmaceutical composition according to claim 18, wherein said proton pump inhibitor pellets comprising enteric coating in an amount of 1% to 20% (w/w) of the total weight of the pellets preferably in an amount of 2% to 10% (w/w).

20. The pharmaceutical composition of any preceding claims, for use in anticoagulant treatment and treating deep vein thrombosis and pulmonary embolism and protecting the gastrointestinal tract from side effects associated with anticoagulant therapy.
